# EUROPÄISCHE PATENTSCHRIFT

(11) **EP 0 658 354 B1**
(45) Veröffentlichungstag und Bekanntmachung des Hinweises auf die Patenterteilung: **28.04.1999**
(21) Anmeldenummer: 94118252.9
(22) Anmeldetag: 19.11.1994
(51) Int. Cl.: A61M 1/36, A61M 5/168

(54) **Dosierungsvorrichtung zum volumetrischen Dosieren eines flüssigen Additivs**
Metering device for volumetric dispensing of a liquid additive
Dispositif de dosage pour délivrer de façon volumétrique un additif liquide

(30) Priorität: 23.11.1993 DE 4339811
(43) Veröffentlichungstag der Anmeldung: 21.06.1995
(73) Patentinhaber: Fresenius AG, 61350 Bad Homburg v.d.H (DE)
(72) Erfinder: Pusinelli, Thomas, D-63674 Altenstadt (DE); Mushoff, Dieter, D-35423 Lich (DE)
(74) Vertreter: Luderschmidt, Schüler & Partner GbR

(56) Entgegenhaltungen:
- EP-A- 0 189 491
- EP-A- 0 361 662
- EP-A- 0 568 265
- US-A- 4 451 255
- US-E- R E33 021

## Beschreibung

Die Erfindung betrifft eine Dosiervorrichtung zum volumetrischen Dosieren eines flüssigen Additivs nach dem Oberbegriff des Patentanspruchs 1. Eine solche Dosiervorrichtung wird insbesondere innerhalb eines Systems zur Sammlung und Retransfusion von autologem Blut verwendet, um dem beispielsweise während einer Operation o.ä. abgesaugtem und zur Retransfusion vorgesehenen Blut eine Antikoagulanzflüssigkeit oder einen Sedimentationsbeschleuniger zuzusetzen.

Ein System zur Sammlung und Retransfusion von autologem Blut ist beispielsweise aus der EP-OS 0 438 703 bekannt. Bei dem bekannten System ist ein Sammelgefäß mit einem Unterdruck beaufschlagt, der dazu verwendet wird, mittels eines an dem Sammelgefäß angeschlossenen Schlauches Blut aus einem Operationsfeld, d.h. dem Körper eines Patienten, abzusaugen. Das abgesaugte Blut, das als Drainageblut bezeichnet wird, fließt durch die Schlauchleitung und passiert eine Mengendosiereinrichtung, bevor es in den Sammelbehälter tropft. Vor der Mengendosiereinrichtung ist stromaufwärts eine Y-förmige Weiche oder Verbindungsstelle angeordnet, in die ein zweiter Schlauch mündet, der mit einem Reservoir für eine Antikoagulanzflüssigkeit und/oder einem Sedimentationsbeschleuniger verbunden ist. Zwischen dem Reservoir für die Antikoagulanzflüssigkeit und der Y-förmigen Weiche befindet sich ein Ventilmittel, das über die erwähnte Mengendosiereinrichtung angesteuert werden kann.

Übliche aus dem Stand der Technik bekannte Dosiereinrichtungen arbeiten nach dem Prinzip der Tropfenzählung, wobei die zu messende bzw. zu überwachende, in einem Schlauch fließende Flüssigkeit einer Tropfkammer zugeleitet wird, die von einer Lichtschranke umgeben ist. Ein in der Tropfkammer fallender Flüssigkeitstropfen unterbricht den Lichtstrahl der Lichtschranke und erzeugt so einen Zählimpuls, der zur weiteren Auswertung einem Mikroprozessor o.ä. zugeleitet werden kann. Nachteilig an dieser Art der Volumenmessung einer fließenden Flüssigkeit ist, daß das Meßergebnis umso ungenauer ist, je größeren Viskositätsschwankungen die zu messende Flüssigkeit unterliegt. Dies liegt darin begründet, daß sich ändernde kinematische Zähigkeit (Viskosität) und sich ändernde Dichte eine Änderung des Volumens eines einzelnen Tropfens mit sich ziehen. Da sich mit der Lichtschranke lediglich die absolute Anzahl von gefallenen Tropfen ermitteln läßt, führt dies dazu, daß sich das tatsächliche volumetrische Mischungsverhältnis in dem Fall ändert, in dem einer bestimmten Anzahl von gefallenen Tropfen ein bestimmtes Volumen an Additiv zugesetzt wird, beispielsweise indem nach einer bestimmten Anzahl von gefallenen, mittels der Lichtschranke ermittelten Tropfen von der Mengendosiervorrichtung ein Signal generiert wird, mittels dessen das in der Schlauchleitung angeordnete, an das Reservoir der Antikoagulanzflüssigkeit sich anschließende Ventilmittel angesteuert und für einen bestimmten Zeitquerschnitt geöffnet wird.

Andererseits ist das Prinzip der Volumenbestimmung mittels Tropfkammer und Lichtschranke billig und einfach zu realisieren und arbeitet - jedenfalls soweit der Lichtschrankenteil betroffen ist - berührungslos, was insbesondere bei Patientenblut verarbeitenden Anlagen und Vorrichtungen aus hygienischen Gründen höchst wünschenswert ist.

Eine Vorrichtung zur dosierten Infusion von mehreren Lösungen ist aus der EP 0 189 491 bekannt. Die Infusionsbehälter sind über Ablaufleitungen, in die jeweils ein Ventil und ein Tropfenzähler geschaltet sind, mit einer zum Patienten führenden Sammelleitung verbunden, in die eine volumetrische Pumpe und ein Tropfendetektor geschaltet sind. Ein Steuergerät steuert die Ventile in den Ablaufleitungen in zyklischer Folge so an, daß jeweils eines der Ventile geöffnet ist, während die übrigen Ventile geschlossen sind.

Der Erfindung liegt die Aufgabe zugrunde, eine Dosiervorrichtung zum volumetrischen Dosieren eines flüssigen Additivs, das einem in einer ersten Schlauchleitung fließenden Fluid über eine zweite Schlauchleitung in einem bestimmten Volumenverhältnis zugesetzt wird, so zu verbessern, daß trotz der Verwendung einer Tropfkammer und einer Lichtschranke zur Tropfenzählung und damit indirekten Volumenermittlung des in der ersten Schlauchleitung fließenden Fluids eine Zudosierung des zuzusetzenden Additivs, insbesondere einer Antikoagulanzflüssigkeit, in einem festen Volumenverhältnis ermöglicht wird, ohne daß die Dosierung durch sich ändernde Viskositäten der in der ersten Schlauchleitung fließenden Flüssigkeit verfälscht wird.

Die Lösung dieser Aufgabe erfolgt erfindungsgemäß mit den im Patentanspruch 1 angegebenen Merkmalen.

Der Erfindung liegt die Erkenntnis zugrunde, daß in dem zu der Tropfkammer führenden Abschnitt der ersten Schlauchleitung, der gegebenenfalls auch in Form eines Rohres ausgebildet sein kann und der als Tropfrohr bezeichnet wird, aufgrund der Kapillarwirkung stets ein bestimmtes Volumen an Fluid verbleibt.

Wird nun die zweite, das zuzusetzende Additiv führende Schlauchleitung mittels der Ventilmittel so geschaltet, daß Additiv in das Tropfrohr fließen kann und wird gleichzeitig die erste Schlauchleitung durch das andere Ventilmittel unterbrochen, so entspricht das in das Tropfrohr nachrückende

Volumen an Additiv dem Volumen des das Tropfrohr verlassenden Tropfens. Wird nun das Mischungsverhältnis zwischen Additiv und primärem Fluid, beispielsweise Blut, in ganzzahligen Vielfachen von Tropfen ausgedrückt, bespielsweise ein Tropfen Additiv pro sieben Tropfen Blut, so hat eine Viskositätsänderung und eine daraus resultierende geänderte Größe eines einzelnen Tropfens keinen Einfluß auf das Gesamtmischungsverhältnis, da beispielsweise bei einem größeren, das Tropfrohr verlassenden Tropfen entsprechend eine größere Menge an Additiv in das Tropfrohr gezogen wird.

Bei einer bevorzugten Ausführungsform der Erfindung sind die Ventilmittel ein 4/2-Wegeventil. Dabei ist das 4/2-Wegeventil bevorzugt mit einer Federvorspannung versehen, so daß das Ventil in seiner Ruhelage die erste Schlauchleitung auf Durchgang schaltet und die zweite, das Additiv führende Schlauchleitung gesperrt ist. Im geschalteten Zustand werden die Verhältnisse dann umgekehrt, so daß die erste Schlauchleitung gesperrt und die zweite Schlauchleitung freigeschaltet ist.

Bei einer weiteren bevorzugten Ausführungsform sind die Ventilmittel ein 3/2-Wegeventil, so daß die zweite Schlauchleitung in dem Ventil indirekt in die erste Schlauchleitung mündet. Anders ausgedrückt sind die Anschlüsse des 3/2-Wegeventils so geschaltet, daß die erste Schlauchleitung in der federzentrierten Ruhelage des Ventils direkt auf das Tropfrohr geschaltet ist, während die zweite, das Additiv führende Schlauchleitung gesperrt ist. Im geschalteten Zustand werden die Verhältnisse wiederum umgekehrt, so daß die zweite, das Additiv führende Schlauchleitung nunmehr direkt auf das Tropfrohr geschaltet und die erste Schlauchleitung gesperrt ist.

In einer weiteren, schaltungstechnisch etwas aufwendigeren Variante sind die Ventilmittel ein 3/3-Wegeventil, das eine federzentrierte Mittelstellung aufweist, in der beide Schlauchleitungen gesperrt sind. Diese zusätzliche Stellung bietet den Vorteil, daß beide Schlauchleitungen gesperrt werden können, beispielsweise wenn ein das abgezogene Blut auffangendes Sammelgefäß ausgetauscht werden soll.

Bei einer weiteren bevorzugten Ausführungsform umfaßt die Zähl- und Steuereinheit der Dosiervorrichtung eine zentrale Recheneinheit (CPU) sowie weiter einen frei programmierbaren Speicher (RAM) und weiterhin Eingabemittel, um einen eine Tropfenanzahl darstellenden Wert N einzugeben. Die zentrale Recheneinheit (CPU) generiert dabei einen Steuerimpuls zur wechselseitigen Ansteuerung der Ventilmittel, wenn die ermittelte Tropfenzahl den im Speicher (RAM) abgelegten Wert N erreicht. Durch die Wahlmöglichkeit für den Wert N wird hiermit dem Bediener eine Möglichkeit gegeben, das volumetrische Mischungsverhältnis, in dem das Additiv zudosiert werden soll, einzustellen.

Weiterhin bevorzugt ist eine Ausführungsform der erfindungsgemäßen Dosiervorrichtung, bei der die Zähl- und Steuereinheit einen frei programmierbaren Speicher (RAM) aufweist, in dem ein Speicherplatz für eine Konstante (A) vorgesehen ist, die der Tropfenanzahl an pro Schaltzyklus der Ventilmittel zuzusetzendem Additiv entspricht. Durch die Wahlmöglichkeit für den Wert A wird eine weitere Möglichkeit geschaffen, auf das Mischungsverhältnis Einfluß zu nehmen und die Vorrichtung auf verschiedene Additive einzustellen.

Weiterhin hat sich als vorteilhaft herausgestellt, daß die Zähl- und Steuereinheit eine zentrale Recheneinheit (CPU) und einen frei programmierbaren Speicher (RAM) aufweist, in dem ein Arbeitsprogramm für die Recheneinheit abgelegt ist, das die Ventilmittel wechselseitig dergestalt steuert, daß nach einer bestimmten Anzahl von Schaltzyklen der Ventilmittel ein sogenannter "Testschaltzyklus" durchgeführt wird, in dem die das Additiv führende, zweite Schlauchleitung so lange durchgeschaltet wird, bis sichergestellt ist, daß die zweite Schlauchleitung bzw. das an dieser angeschlossene Reservoir noch einen Vorrat an Additiv enthält. Zu diesem Zweck wird in der geschalteten Stellung des Ventils eine so große Anzahl von Tropfen zugelassen, daß das durch die Lichtschranke erfaßte, abgeflossene Volumen an Additiv dem Volumen des Tropfrohres und eventuell in den Ventilmitteln enthaltenen Toträumen auf jeden Fall entspricht, auch dann, wenn aufgrund von Viskositätseinflüssen nur sehr kleine Tropfen fallen sollten.

Weiterhin ist bevorzugt vorgesehen, daß die Dosiervorrichtung Einwegteile umfaßt, die nach Gebrauch bzw. Kontamination mit Blut entsorgt werden. Zu diesen als Wegwerfteilen ausgeführten Einzelteilen können insbesondere die beiden Schlauchleitungen, die beiden Ventilmittel, das Tropfrohr und die Tropfkammer gehören. Auf diese Weise wird eine hygienisch einwandfreie handhabbare Vorrichtung geschaffen, bei der die teuren Teile, wie die Lichtschranke und die Zähl- und Steuereinheit, die keiner Verschmutzung und keinem Verschleiß unterworfen sind, erhalten bleiben, hingegen die mit Blut in Berührung kommenden Teile schnell und einfach ausgetauscht werden können, was insbesondere im Krankenhausbetrieb einer schnellen und effizienten Handhabung sehr entgegenkommt.

Um die Austauschbarkeit insbesondere einer als Einwegteil ausgebildeten Tropfkammer einfach zu gestalten, können die Tropfkammer und die Lichtschranke vorzugsweise so ausgebildet sein, daß die Tropfkammer in der entsprechend ausgebildeten Lichtschranke verriegelt werden kann. Dies geschieht vorzugsweise dadurch, daß die Tropfkammer formschlüssig in dem die Lichtschranke enthaltenden Bauteil einrastbar ist, d.h., daß in dem Gehäuse der Lichtschranke eine Aussparung vorgesehen ist, in der die teilweise durchsichtig ausgebildete Tropfkammer eingerastet werden kann. Die Tropfkammer kann insbesondere aus einem Kunststoff in Spritzgießtechnik hergestellt sein.

Die Erfindung wird im folgenden anhand zweier in der Zeichnung dargestellter bevorzugter Ausführungsbeispiele näher erläutert.

In der Zeichnung zeigen:
- **Fig. 1**: eine schematische Darstellung einer ersten Ausführungsform, und
- **Fig. 2**: eine schematische Darstellung einer zweiten Ausführungsform einer Dosiervorrichtung gemäß der Erfindung.

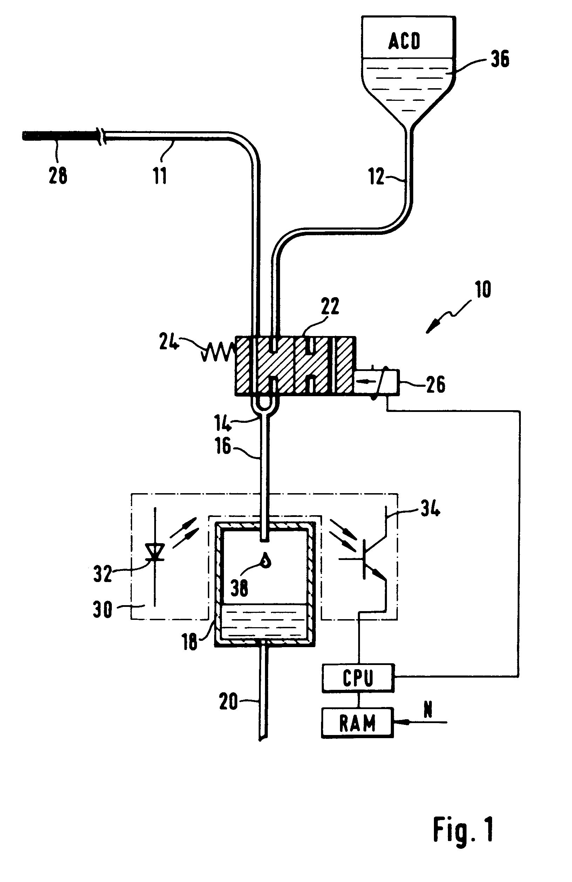

Die in Fig. 1 dargestellte Ausführungsform einer erfindungsgemäßen Dosiervorrichtung ist allgemein mit dem Bezugszeichen 10 bezeichnet. Sie weist eine erste Schlauchleitung 11 und eine zweite Schlauchleitung 12 auf. Die beiden Schlauchleitungen 11 und 12 treffen sich an einer Y-förmigen Einmündungsstelle 14 und gehen in ein Tropfrohr 16 über. An das Tropfrohr schließt sich eine Tropfkammer 18 an, die über eine Abzugsleitung 20 mit einem Unterdruck beaufschlagt wird. In die beiden Schlauchleitungen 11 und 12 sind zusammengefaßte Ventilmittel in Form eines 4/2-Wegeventils 22 eingefügt. Das 4/2-Wegeventil wird durch eine Druckfeder 24 in einer Ruhestellung gehalten, in der die erste Schlauchleitung 11 auf Durchgang geschaltet ist, während die zweite Schlauchleitung 12 gesperrt ist. Das 4/2-Wegeventil 22 kann elektrisch mittels eines Solenoids 26 betätigt werden, so daß in der dann eingenommenen Schaltstellung die erste Schlauchleitung 11 gesperrt ist, während die zweite Schlauchleitung 12 mit dem Tropfrohr 16 verbunden ist.

Bei dem in Fig. 1 dargestellten Zustand, bei dem sich das Ventil 22 in der Ruhelage befindet, ist die erste Schlauchleitung 11 auf Durchgang geschaltet. Der in der Tropfkammer 18 wirkende Unterdruck, der über die Abzugsleitung 20 aufgebracht wird, wirkt über das Tropfrohr 16 und das Ventil 22 bis in die Schlauchleitung 11 hinein, die einen perforierten Endabschnitt 28 aufweist. Der Endabschnitt 28 der Schlauchleitung 11 steckt beispielsweise in einer Operationswunde und saugt dort Blut und/oder Wundfüssigkeit ab. Das abgesaugte Blut wird durch die Schlauchleitung 11 und das auf Durchgang geschaltete Ventil 22 gefördert und tropft am Ende des Tropfrohres 16 in die Tropfkammer 18.

Die herabfallenden Tropfen unterbrechen den quer zur Tropfrichtung ausgesandten Lichtstrahl einer Lichtschranke 30, die die Tropfkammer umgebend ausgebildet ist. Die Lichtschranke 30 ist in herkömmlicher Weise aufgebaut und umfaßt beispielsweise als Lichtquelle eine Licht emittierende Diode (LED) 32, und als Empfängerelement einen Fototransistor 34. Durch einen fallenden Tropfen 38 wird der von der Leuchtdiode 32 beleuchtete Fototransistor 34 verdunkelt, wodurch ein Impuls erzeugt wird, der einer zentralen Recheneinheit (CPU) zugeleitet wird. In der Recheneinheit werden die durch die herabfallenden Tropfen 38 erzeugten Impulse aufaddiert und mit einem in einem frei programmierbaren Speicher (RAM) abgelegten Sollwert N verglichen. Der eingestellte Sollwert kann beispielsweise 7 betragen. Nach 7 herabgefallenen Tropfen wird von der zentralen Recheneinheit (CPU) ein Steuerimpuls generiert, der entsprechend verstärkt dem Solenoid 26 des Ventils 22 zugeleitet wird, woraufhin dieses schaltet und die Schlauchleitung 11 sperrt, hingegen aber die Schlauchleitung 12 freigibt. Am oberen Ende der Schlauchleitung 12 ist ein Reservoir 36 angeordnet, das eine Antikoagulanzflüssigkeit (ACD) enthält, sowie gegebenenfalls weiter Zusätze eines Senkungsbeschleunigers, der das Volumen der in dem Blut enthaltenen Erythrozytenaggregate erhöht und so eine Sedimentation, d.h. ein Trennen von Blutplasma und gegebenenfalls enthaltener Wundflüssigkeit, Lymphwasser o.ä. beschleunigt.

Die Schlauchleitung 12 ist durch das nunmehr geöffnete Ventil 22 direkt auf das Tropfrohr 16 geschaltet, das zu diesem Zeitpunkt wegen der Kapillarwirkung noch mit Blut gefüllt ist. Sobald ein weiterer Tropfen 38 am unteren Ende des Tropfrohres 16 fällt, wird am oberen Ende des Tropfrohres über das durchgeschaltete Ventil 22 ein entsprechendes Volumen an ACD (Antikoagulanzflüssigkeit) aus der Schlauchleitung 12 nachgezogen. Da das am oberen Ende des Tropfrohres ersetzte Volumen dem Volumen genau entspricht, das in Tropfenform am unteren Ende des Tropfrohres abgegeben worden ist, hat die Größe des das Tropfrohr verlassenden Tropfens keinen Einfluß auf das Mischungsverhältnis, das beispielsweise im vorliegenden Beispiel in Volumenteilen 7:1 beträgt.

Nachdem mittels der Lichtschranke und der zentralen Recheneinheit (CPU) ein weiterer herabfallender Tropfen 38 detektiert worden ist, erzeugt die CPU einen weiteren Steuerimpuls, mit dem der Erregerstrom des Solenoids 26 abgeschaltet wird, woraufhin die Druckfeder 24 das 4/2-Wegeventil in seine Ruhelage drückt, so daß die Schlauchleitung 11 auf Durchgang geschaltet ist und weiteres Blut durch den Drainageschlauch 11 gezogen wird.

Um sicherzustellen, daß das Reservoir 36 für die Antikoagulanzflüssigkeit noch gefüllt ist, kann vorgesehen sein, nach einer bestimmten Anzahl von Schaltwechseln das Schaltventil 22 nicht nur für die Zeitdauer eines herabfallenden Tropfens 38 in der Schaltstellung zu halten, sondern das Ventil für eine Anzahl von herabfallenden Tropfen in der die Schlauchleitung 12 durchschaltenden Schaltstellung zu halten, die so bemessen ist, daß das Gesamtvolumen der herabfallenden Tropfen geringfügig größer ist als das Innenvolumen des Tropfrohres 16 plus eventueller Toträume im Ventil 22. Auf diese Weise kann sichergestellt werden, daß noch Antikoagulanzflüssigkeit im Reservoir 36 enthalten ist.

Zur Steuerung dieser Routine kann ein entsprechendes die CPU steuerndes Programm im Speicher (RAM) abgelegt sein.

Um einer erhöhten Konzentration der Antikoagulanzflüssigkeit im Blut zu begegnen, die sich durch eine solche Zudosierung von Antikoagulanzflüssigkeit zu Prüfzwecken ergäbe, kann vorgesehen sein, den Wert N für die normalen Schaltzyklen entsprechend zu erhöhen, so daß das Gesamtmischungsverhältnis zwischen Blut und Antikoagulanzflüssigkeit im Auffangbehälter, der sich an die Tropfkammer anschließt, nach einer endlichen Zahl von Schaltzyklen des Ventils trotz der zusätzlichen Beigabe von Antikoagulanzflüssigkeit während des "Testzyklus" wieder dem ursprünglich beabsichtigten Mischungsverhältnis entspricht.

Dabei ist die maßgeblichen Einflußgröße die Anzahl an Tropfen, die notwendig ist, um ein Volumen abzumessen, das sicher größer ist als das Volumen des Tropfrohres plus dem Volumen eventueller Toträume in dem Ventil 22, und zwar auch in dem ungünstigsten Fall, daß die kleinstdenkbare Tropfengröße produziert wird, weil beispielsweise die Viskosität des Blutes einen ungünstigen Einfluß hat. Ein über diese Anzahl hinaus noch fallender Tropfen zeigt dann an, daß noch ACD vorhanden ist.

Auf diese Anzahl kann durch die Dimensionierung des Tropfrohres Einfluß genommen werden. Weiterhin ist die Anzahl von Schaltzyklen variabel, die durchlaufen werden, bevor ein "Testschaltzyklus" vorgenommen wird, im Verlaufe dessen das Schaltventil 22 länger als nur für einen fallenden Tropfen in der Schaltstellung, d.h. bei durchgeschalteter Antikoagulanzflüssigkeits-Leitung 12, verbleibt.

Ist beispielsweise ein Mischungsverhältnis von 7:1 angestrebt, so wird bei normalem Rhythmus, d.h. ohne Testzyklus, das Ventil 22 für sieben herabfallende Tropfen 38 in der Ruhestellung verbleiben und schaltet dann für einen Tropfen in die Schaltstellung.

Nimmt man nun an, daß jeder zehnte Schaltzyklus ein Testschaltzyklus sein soll, bei dem insgesamt elf Tropfen durch die Tropfkammer fallen, während Antikoagulanzflüssigkeit durch das sich in der Schaltstellung befindende Ventil 22 gezogen wird, so ist die Anzahl der Tropfen, die fallen, während sich das Schaltventil jeweils in der Ruhestellung befindet, d.h. während die Schlauchleitung 11 auf Durchgang geschaltet ist, auf vierzehn zu erhöhen. Nach zehn kompletten Schaltzyklen sind dann insgesamt einhundertundvierzig Tropfen Blut in das Tropfrohr nachgerückt, während während der neun normalen Schaltzyklen neun Tropfen Antikoagulanzflüssigkeit und im zehnten Schaltzyklus elf Tropfen Antikoagulanzflüssigkeit zudosiert worden sind. Das gesamte Mischungsverhältnis beträgt daher 140:20, d.h. 7:1, wie ohne Testschaltzyklus.

Aufgrund der leicht nachvollziehbaren Zusammenhänge lassen sich bei entsprechender Dimensionierung des Tropfrohres oder der Anzahl von normalen Schaltzyklen bzw. der Mischungsverhältnisse andere Zahlenverhältnisse finden, die eine Verwendung eines beschriebenen Testschaltzyklus gestatten, ohne das Mischungsverhältnis zu verändern. Erfindungsgemäß hat eine wechselnde Tropfengröße aufgrund sich ändernder Viskositäten keinen Einfluß auf das Mischungsverhältnis.

Erfindungsgemäß ist vorgesehen, daß die Tropfkammer 18 als Einwegteil ausgebildet ist, die mit ebenfalls aus Kunststoff gefertigten Schläuchen 11 und 12 bzw. einem aus einfachen Kunststoffbauteilen gefertigten Ventil zusammengekoppelt ist.

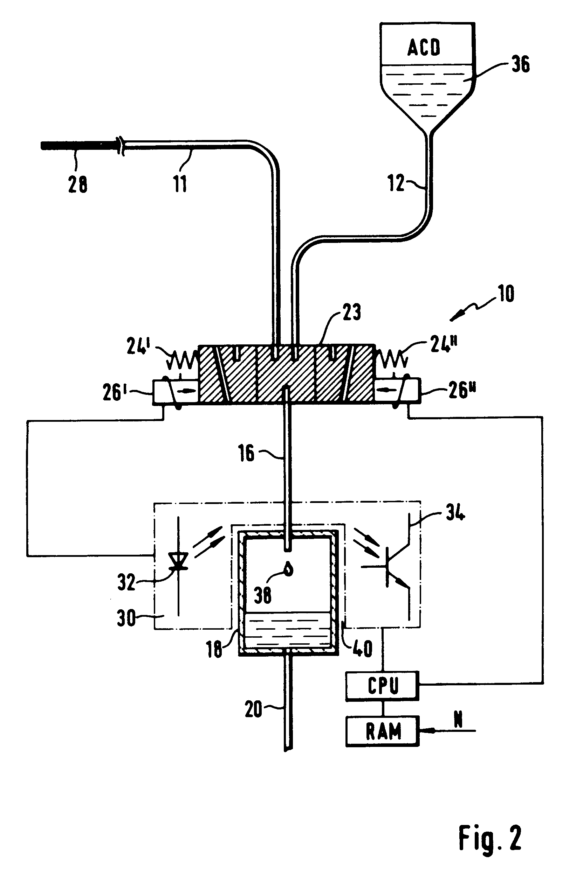

In Fig. 2 ist eine schaltungstechnisch etwas aufwendigere Variante der erfindungsgemäßen Dosiervorrichtung dargestellt, bei der anstelle eines 4/2-Wegeventils ein 3/3-Wegeventil verwendet wird. Die übrigen Bauteile und Elemente entsprechen der in Fig. 1 dargestellten Ausführungsform, wobei gleiche Teile gleiche Bezugszeichen aufweisen, so daß auf eine Wiederholung der diesbezüglichen Beschreibung verzichtet wird.

Das 3/3-Wegeventil 23 in Fig. 2 weist zwei Druckfedern 24' und 24'' auf, mittels derer es mittenzentriert ist. In der dargestellten Ruhelage des 3/3-Wegeventils 23 sind sowohl die Schlauchleitung 11 wie auch die Schlauchleitung 12 gesperrt, so daß keinerlei Flüssigkeit zu dem am dritten Anschluß des 3/3-Wegeventils angeschlossenen Tropfrohr gelangen kann. Im Betrieb wird von der CPU ein Schaltsignal generiert, das entsprechend verstärkt einem ersten Solenoid 26' zugeleitet wird, so daß das 3/3-Wegeventil 23 in eine erste Schaltstellung geschaltet wird, in der die Drainageleitung bzw. Schlauchleitung 11 mit dem Tropfrohr 16 verbunden ist, während die die Antikoagulanzflüssigkeit führende Schlauchleitung 12 weiterhin gesperrt bleibt. Bei dieser Ausführungsform der Erfindung sind also die beiden Schlauchleitungen 11 und 12 nicht direkt miteinander an einer Einmündungsstelle gekoppelt, sondern sind indirekt miteinander durch das 3/3-Wegeventil 23 verbunden. Nach einer festgelegten Anzahl N generiert die CPU erneut einen Schaltimpuls, der diesmal entsprechend verstärkt dem Solenoid 26 zugeleitet wird, woraufhin das 3/3-Wegeventil in eine zweite Schaltstellung gedrückt wird, in der die Schlauchleitung 12 mit dem Tropfrohr 16 verbunden ist, so daß ein weiterer fallender Tropfen 38 Antikoagulanzflüssigkeit in das Tropfrohr 16 zieht.

Anhand von Fig. 2 wird ersichtlich, daß sich die erfindungsgemäße Dosierungsvorrichtung auch mit einem 3/2-Wegeventil realisieren läßt. Denkt man sich in Fig. 2 die mittlere, federzentrierte Ruhestellung des 3/3-Wegeventils weg, so verbleiben zwei Schaltstellungen eines 3/2-Wegeventils. Dabei würde bei einer solchen Ausführungsform die Druckfeder 24'' und das linke Solenoid 26' entfallen, so daß die verbleibende Druckfeder 24' das 3/2-Wegeventil in eine Ruhestellung drückt, in der die Schlauchleitung 11 mit dem Tropfrohr 16 verbunden ist. Nach einer entsprechenden Anzahl von gefallenen Tropfen N generiert die CPU einen entsprechenden Schaltimpuls, der dazu verwendet wird, einen Erregerstrom auf das Solenoid 26'' zu legen, so daß das 3/2-Wegeventil an eine Schaltstellung geschaltet wird, in der die Antikoagulanzflüssigkeit führende Schlauchleitung 12 auf das Tropfrohr 16 geschaltet ist.

Wie in den Figuren weiterhin angedeutet, ist die Lichtschranke 30 in einem Gehäuse eingebaut, das eine nutartige Aussparung 40 aufweist. In diese Aussparung 32 kann die entsprechend ausgebildete Tropfkammer 18 formschlüssig eingerastet werden. Die Abflußleitung 20, die Tropfkammer 18, das Tropfrohr 16, das Ventil 22 bzw. 23 und die beiden Schlauchleitungen 11 und 12 können als Einwegteile ausgebildet sein, die sich einfach austauschen lassen, wenn das Gerät, d.h. die wiederzuverwertenden Teile, wie insbesondere Lichtschranke 30, CPU und RAM beispielsweise bei einer anderen Operation verwendet werden sollen.

Die erfindungsgemäße Dosiervorrichtung erlaubt eine genaue volumetrische Zudosierung eines Additivs, ohne daß eine sich ändernde Tropfengröße von mittels einer Lichtschranke erfaßter und gezählter Tropfen einen Einfluß auf die Genauigkeit der Dosierung hat.

### Bezugszeichenliste

- 10: Dosiervorrichtung
- 11: erste Schlauchleitung
- 12: zweite Schlauchleitung
- 14: Einmündungsstelle (von 12 in 11)
- 16: Tropfrohr
- 18: Tropfkammer
- 20: Abzugleitung
- 22: 4/2-Wegeventil
- 23: 3/3-Wegeventil
- 24, 24', 24'': Druckfeder
- 26, 26', 26'': Solenoide
- 28: perforierter Endabschnitt (von 11)
- 30: Lichtschranke
- 32: Leuchtdiode
- 34: Fototransistor
- 36: Reservoir
- 38: Tropfen
- 40: Aussparung (in 30)

## Patentansprüche

1. Dosiervorrichtung zum volumetrischen Dosieren eines flüssigen Additivs, das einem in einer ersten Schlauchleitung (11) fließenden Fluid über eine zweite Schlauchleitung (12) in einem bestimmten Volumenverhältnis zugesetzt wird, mit
elektrisch ansteuerbaren Ventilmitteln (22, 23), die in beide Schlauchleitungen (11, 12) eingefügt sind und wechselweise jeweils nur eine der beiden Schlauchleitungen zu einem stromab sich erstreckenden gemeinsamen Schlauch- oder Rohrabschnitt (16) durchgängig schalten,
einer Zähl- und Steuereinheit (CPU, RAM) und
einer sich an den gemeinsamen Schlauch- oder Rohrabschnitt (16) anschließenden Tropfkammer (18) mit einer Lichtschranke (30), deren Wirkungsrichtung quer zur Fallstrecke der Tropfen angeordnet ist,
dadurch gekennzeichnet,
daß die in der Tropfkammer (18) fallenden Tropfen von der Zähleinheit (CPU, RAM) zählbar und die Ventilmittel (22, 23) von der Steuereinheit (CPU, RAM) nach einer bestimmten Anzahl von Tropfen wechselweise ansteuerbar sind, so daß der gemeinsame Schlauch- oder Rohrabschnitt (16) mit der ersten oder zweiten Schlauchleitung (11, 12) wechselweise verbunden ist.

2. Dosiervorrichtung nach Anspruch 1, dadurch gekennzeichnet, daß die Ventilmittel ein 4/2-Wegeventil (22) sind.

3. Dosiervorrichtung nach Anspruch 1, dadurch gekennzeichnet, daß die Ventilmittel ein 3/2-Wegeventil sind.

4. Dosiervorrichtung nach einem der Ansprüche 2 oder 3, dadurch gekennzeichnet, daß das Ventilmittel (22) federbelastet ist, wobei in der Ruhestellung die erste Schlauchleitung (11) auf Durchgang geschaltet ist.

5. Dosiervorrichtung nach Anspruch 1, dadurch gekennzeichnet, daß die Ventilmittel ein 3/3-Wegeventil (23) sind, das eine federzentrierte Mittelstellung aufweist, in der beide Schlauchleitungen (11, 12) gesperrt sind.

6. Dosiervorrichtung nach einem der vorhergehenden Ansprüche, dadurch gekennzeichnet, daß die Zähl- und Steuereinheit eine zentrale Recheneinheit (CPU) umfaßt, sowie weiter einen frei programmierbaren Speicher (RAM), und weiterhin Eingabemittel, um einen eine Tropfenanzahl darstellenden Wert (N) einzugeben, und daß die zentrale Recheneinheit (CPU) einen Steuerimpuls zur wechselseitigen Ansteuerung der Ventilmittel (22; 23) generiert, wenn die ermittelte Tropfenzahl den im Speicher (RAM) abgelegten Wert (N) erreicht.

7. Dosiervorrichtung nach einem der vorhergehenden Ansprüche, dadurch gekennzeichnet, daß die Zähl- und Steuereinheit einen frei programmierbaren Speicher (RAM) aufweist, in dem ein Speicherplatz für eine Konstante (A) vorgesehen ist, die der Tropfenanzahl an pro Schaltzyklus der Ventilmittel zuzusetzendem Additiv entspricht.

8. Dosiervorrichtung nach einem der vorhergehenden Ansprüche, dadurch gekennzeichnet, daß die Zähl- und Steuereinheit eine zentrale Recheneinheit (CPU) und einen frei programmierbaren Speicher (RAM) aufweist, in dem ein Arbeitsprogramm für die Recheneinheit abgelegt ist, das die Ventilmittel (22; 23) wechselseitig dergestalt steuert, daß nach einer bestimmten Anzahl von Schaltzyklen der Ventilmittel ein Schaltzyklus durchgeführt wird, in dem die das Additiv führende, zweite Schlauchleitung (12) so lange durchgeschaltet wird, bis das durch die Lichtschranke (30) erfaßte, abgeflossene Volumen an Additiv dem Volumen des Schlauch- oder Rohrabschnitts (16) und eventuell in den Ventilmitteln (22; 23) enthaltenen Toträumen entspricht (Testschaltzyklus).

9. Dosiervorrichtung nach einem der vorhergehenden Ansprüche, dadurch gekennzeichnet, daß die beiden Schlauchleitungen (11, 12), die Ventilmittel (22; 23), der Schlauch- oder Rohrabschnitt (16) und die Tropfkammer (18) als Einwegteile ausgebildet sind.

10. Dosiervorrichtung nach Anspruch 9, dadurch gekennzeichnet, daß die Tropfkammer (18) und die Lichtschranke (30) so ausgebildet sind, daß die als Einwegteil ausgebildete Tropfkammer in der entsprechend ausgebildeten Lichtschranke verriegelt werden kann.

11. Dosiervorrichtung nach Anspruch 10, dadurch gekennzeichnet, daß die Tropfkammer (18) formschlüssig in dem die Lichtschranke (30) enthaltenen Bauteil einrastbar ist.

## Claims

1. A metering device for the volumetric dispensing of a liquid additive which is added to a fluid flowing through a first pipe (11) in a specific volumetric ratio and through a second pipe (12), and with
electrically operable valve means (22, 23) incorporated into both pipes (11, 12) and in each case and alternately switching only one of the two pipes through to a common tube or pipe portion (16) on the downstream side,
a counting and control unit (CPU, RAM) and
a dropping chamber (18) with a light barrier (30) adjacent the common tube or pipe portion (16) and the directional action of which is crosswise to the path through which the drops fall,
characterised in that
the drops falling in the dropper chamber (18) can be counted by the counting unit (CPU, RAM) and the valve means (22, 23) can be alternately operated by the control unit (CPU, RAM) after a predetermined number of drops so that the common tube or pipe portion (16) is alternately connected to the first or second pipe (11, 12).

2. A metering device according to claim 1, characterised in that the valve means are a 4/2-way valve (22).

3. A metering device according to claim 1, characterised in that the valve means are a 3/2-way valve.

4. A metering device according to one of claims 2 or 3, characterised in that the valve means (22) are spring-loaded, whereby in the inoperative position, the first pipe (11) is switched to through-flow.

5. A metering device according to claim 1, characterised in that the valve means are a 3/3-way valve (23) having a spring-centred central position in which both pipes (11, 12) are closed.

6. A metering device according to one of the preceding claims, characterised in that the counting and control unit comprises a central processing unit (CPU) and furthermore a freely programmable memory (RAM) and furthermore input means in order to key in a value (N) representing a number of drops and in that the central processing unit (CPU) generates a control pulse for alternately actuating the valve means (22, 23) when the ascertained number of drops reaches the value (N) stored in the memory (RAM).

7. A metering device according to one of the preceding claims, characterised in that the counting and control unit comprises a freely programmable memory (RAM) in which a memory site is provided for a constant (A) which corresponds to the number of drops of additive to be added per switching cycle of the valve means.

8. A metering device according to one of the preceding claims, characterised in that the counting and control unit comprises a central processing unit (CPU) and a freely programmable memory (RAM) in which there is stored for the processing unit a working programme which alternately operates the valve means (22, 23) in such a way that after a definite number of switching cycles of the valve means a switching cycle is carried out in which the second pipe (12) carrying the additive is switched to through-flow until such time as the volume of additive which has flowed through and which has been ascertained by the light barrier (30) corresponds to the volume of the tube or pipe portion (16) and possibly dead spaces contained in the valve means (22, 23).

9. A metering device according to one of the preceding claims, characterised in that the two pipes (11, 12), the valve means (22, 23), the tube or pipe portion (16) an the dropper chamber (18) are constructed as disposable parts.

10. A metering device according to claim 9, characterised in that the dropper chamber (18) and the light barrier (30) are so constructed that the dropper chamber which is constructed as a disposable part can be locked in the correspondingly constructed light barrier.

11. A metering device according to claim 10, characterised in that the dropper chamber (18) can be snap-fitted positively in the component containing the light barrier (30).

## Revendications

1. Dispositif de dosage pour le dosage volumétrique d'un additif liquide qui est ajouté, dans une proportion en volume déterminée, à un fluide liquide dans une première conduite flexible (11) par l'intermédiaire d'une seconde conduite flexible (12), comportant
des organes de soupape (22, 23) qui sont commandables électriquement, sont insérés dans les deux conduites flexibles (11, 12) et qui actionnent en général alternativement, respectivement uniquement l'une des deux conduites flexibles vers une section commune de tube flexible ou de tuyau (16) s'étendant en aval,
une unité de comptage et de commande (CPU, RAM) et
une chambre compte-gouttes (18) se raccordant à la section commune de tube flexible ou de tuyau (16) et présentant une barrière photoélectrique (30) dont le sens d'action est disposé transversalement à la trajectoire de chute des gouttes,
caractérisé en ce que
les gouttes tombant dans la chambre compte-gouttes (18) peuvent être comptées par l'unité de comptage (CPU, RAM) et en ce que les organes de soupape (22, 23) peuvent être alternativement commandés par l'unité de commande (CPU, RAM) après un nombre déterminé de gouttes de telle sorte que la section commune de tube flexible ou de tuyau (16) est reliée alternativement à la première ou à la seconde conduite flexible (11, 12).

2. Dispositif de dosage selon la revendication 1, caractérisé en ce que les organes de soupape sont une soupape à 4/2 canaux (22).

3. Dispositif de dosage selon la revendication 1, caractérisé en ce que les organes de soupape sont une soupape à 3/2 canaux.

4. Dispositif de dosage selon la revendication 2 ou 3, caractérisé en ce que l'organe de soupape (22) est à ressort, la première conduite flexible (11) étant commutée sur passage lorsqu'elle est en position de repos.

5. Dispositif de dosage selon la revendication 1, caractérisé en ce que les organes de soupape sont une soupape à 3/3 canaux (23) qui présente une position médiane ajustée par ressort dans laquelle les deux conduites flexibles (11, 12) sont bloquées.

6. Dispositif de dosage selon l'une quelconque des revendications précédentes, caractérisé en ce que l'unité de comptage et de commande comprend une unité de calcul centrale (CPU), une mémoire librement programmable (RAM) et en outre, des moyens d'entrée pour entrer une valeur (N) représentant un nombre de gouttes et en ce que l'unité de calcul centrale (CPU) génère une impulsion de commande pour la commande alternative des organes de soupape (22, 23) quand le nombre de gouttes déterminé atteint la valeur (N) déposée dans la mémoire (RAM).

7. Dispositif de dosage selon l'une quelconque des revendications précédentes, caractérisé en ce que l'unité de comptage et de commande présente une mémoire (RAM) librement programmable dans laquelle il est prévu un emplacement de mémoire pour une constante (A) qui correspond au nombre de gouttes d'additif devant être ajouté par cycle de commutation des organes de soupape.

8. Dispositif de dosage selon l'une quelconque des revendications précédentes, caractérisé en ce que l'unité de comptage et de commande présente une unité de calcul centrale (CPU) et une mémoire (RAM) librement programmable dans laquelle il est déposé un programme de travail pour l'unité de calcul qui commande alternativement les organes de soupape (22, 23) de telle sorte qu'après un nombre déterminé de cycles de commutation des organes de soupape est effectué un cycle de commutation dans lequel la seconde conduite flexible (12) amenant l'additif est commutée jusqu'à ce que le volume d'additif écoulé et enregistré par la barrière photoélectrique corresponde au volume de la section de tube flexible ou de tuyau (16) et éventuellement, aux espaces morts contenus dans les organes de soupape (22, 23) (cycle de commutation de test).

9. Dispositif de dosage selon l'une quelconque des revendications précédentes, caractérisé en ce que les deux conduites flexibles (11, 12), les organes de soupape (22, 23), la section de tube flexible ou de tuyau (16) et la chambre compte-gouttes (18) sont réalisées comme pièces jetables.

10. Dispositif de dosage selon la revendication 9, caractérisé en ce que la chambre compte-gouttes (18) et la barrière photoélectrique (30) sont réalisées de telle sorte que la chambre compte-gouttes conçue comme une pièce jetable peut être verrouillée dans la barrière photoélectrique conçue de manière correspondante.

11. Dispositif de dosage selon la revendication 10, caractérisé en ce que la chambre compte-gouttes (18) peut être encliquetée de manière ajustée dans le composant contenant la barrière photoélectrique (30).
